# EUROPÄISCHE PATENTANMELDUNG

(11) **EP 0 916 662 A2**
(43) Veröffentlichungstag der Anmeldung: **19.05.1999**
(21) Anmeldenummer: 98119589.4
(22) Anmeldetag: 16.10.1998
(51) Int. Cl.: C07D 307/62, A61K 7/42

(54) **Ascorbylsorbate**

(30) Priorität: 14.11.1997 DE 19750528
(71) Anmelder: BASF AKTIENGESELLSCHAFT, 67056 Ludwigshafen (DE)
(72) Erfinder: Streicher, Harald Dr., 67069 Ludwigshafen (DE); von dem Bussche-Hünnefeld, Linda Dr., 68623 Lampertheim (DE); Westenfelder, Horst, 67435 Neustadt (DE); Wekel, Hans-Ulrich Dr., 67158 Ellerstadt (DE)

(57) **Zusammenfassung**

Ascorbylsorbate der allgemeinen Formel I, 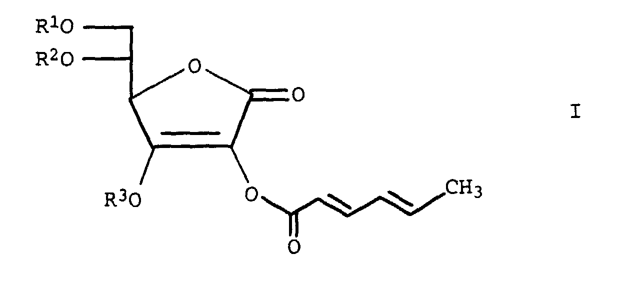 in der die Variablen unabhängig voneinander folgende Bedeutung haben:
R¹ und R²
   Wasserstoff, C₁-C₁₂-Alkyl, C₁-C₂₀-Acyl;
   wobei R¹ und R² gemeinsam mit den Sauerstoffatomen an die sie gebunden sind und den an den Sauerstoffatomen gebundenen Kohlenstoffatomen einen gegebenenfalls substituierten Heterozyklus bilden können;
R³ Wasserstoff oder ein Kation, ausgewählt aus der Gruppe, bestehend aus Alkali- und Erdalkalimetallen.

## Beschreibung

Die UV-Schädigung der Haut ist zum Teil durch die Einwirkung von freien Radikalen zu erklären. Topisch aufgebrachte Ascorbinsäure schützt die Haut vor UV-Schädigung (D. Darr, Br. J. Dermatol, 127 (1992) 247-253), da Ascorbinsäure als Antioxidans gegen freie Radikale wirkt.

Die Erfahrung der Kosmetikindustrie zeigt aber, daß Ascorbinsäure in den meisten Formulierungen zu instabil ist. Deshalb werden Derivate der Ascorbinsäure eingesetzt, welche eine höhere Stabilität in Formulierungen aufweisen, aber immer noch in der Lage sind, Ascorbinsäure freizusetzen. Als Beispiel hierfür ist das L-Ascorbinsäure-2-O-D-glucosid zu nennen, das jedoch für den Einsatz in der Kosmetik häufig nicht lipophil genug ist.

Weitere wichtige Radikalfänger sind Tocopherol (Vitamin E) und Sorbinsäure, die im Kosmetiksektor ebenfalls vielfach eingesetzt werden.

So beschreibt EP-A-0 313 304 die Verwendung von Tocopherylsorbat als Photoprotektor.

Während UV-Filter auf der Haut bleiben und so ein Eindringen der Strahlung in die Haut verhindern sollen, wirkt Tocopherylsorbat der Bildung von freien Radikalen in der Haut entgegen. Da der Wirkstoff in der Haut vorliegt, läßt er sich nicht abreiben oder waschen und bietet so einen längeren Schutz, als die klassischen UV-Filter.

Es bestand nun die Aufgabe, neue Radikalfänger für die kosmetische und pharmazeutische Anwendung bereit zu stellen, die oxidationsstabil sind und über ein breites Eigenschaftsprofil hinsichtlich ihrer Lipophilie verfügen.

Diese Aufgabe wurde erfindungsgemäß gelöst durch Ascorbylsorbate der allgemeinen Formel I, 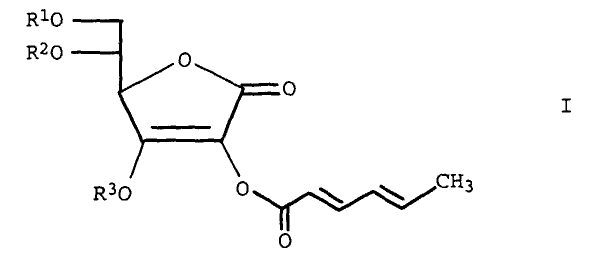 in der die Variablen unabhängig voneinander folgende Bedeutung haben:
R¹ und R²
   Wasserstoff, C₁-C₁₂-Alkyl, C₁-C₂₀-Acyl;
   wobei R¹ und R² gemeinsam mit den Sauerstoffatomen an die sie gebunden sind und den an den Sauerstoffatomen gebundenen Kohlenstoffatomen einen gegebenenfalls substituierten Heterozyklus bilden können;
R³ Wasserstoff oder ein Kation, ausgewählt aus der Gruppe, bestehend aus NH₄⁺, Alkali- und Erdalkalimetallkationen.

Bevorzugt sind solche Verbindungen der Formel I, in der die Variablen unabhängig voneinander folgende Bedeutung haben:
R¹ und R²
   Wasserstoff, C₆-C₂₀-Acyl;
R³ Wasserstoff oder ein Kation, ausgewählt aus der Gruppe, bestehend aus NH₄⁺, Alkali- und Erdalkalimetallkationen.

Besonders bevorzugt sind solche Verbindungen der Formel I, in der die Variablen unabhängig voneinander folgende Bedeutung haben:
R¹ und R²
   Wasserstoff, 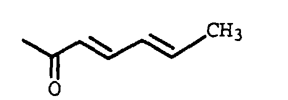
R³ Wasserstoff oder ein Kation, ausgewählt aus der Gruppe, bestehend aus NH₄⁺, Alkali- und Erdalkalimetallkationen;

Bei den erfindungsgemäßen Ascorbylsorbaten der Formel I sind als Acylreste für R¹ und R² verzweigte oder unverzweigte, gesättigte oder ungesättigte, gegebenenfalls mehrfach ungesättigte C₁-C₂₀-Acylketten zu verstehen.

Beispiele hierfür sind Acylreste der Ameisen-, Essig-, Propion-, n-Butter-, iso-Butter-, Sorbin-, n-Valerian-, iso-Valerian-, Capron-, Capryl-, Caprin-, Undecan-, Laurin-, Tridecan-, Mysterin-, Pentadecan-, Palmitin-, Palmitolein-, Stearin-, Öl-, Linol-, Linolen-, Nonadecan- und Arachidonsäure.

Bevorzugt sind die Acylreste der Sorbinsäure sowie die der langkettigen Fettsäuren mit C₁₀ bis C₂₀ Kohlenstoffketten, besonders bevorzugt Acylreste der Sorbin-, Laurin-, Palmitin-, Palmitolein-, Stearin-, Öl- und Linolsäure.

Als Alkylreste R¹ und R² seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl genannt.

Besonders bevorzugte Alkylreste sind C₁-C₆-Alkylketten, insbesondere Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl.

Die Reste R¹ und R² können gemeinsam mit den Sauerstoffatomen an die sie gebunden sind und den an den Sauerstoffatomen gebundenen Kohlenstoffatomen einen gegebenenfalls substituierten Heterozyklus bilden. Darunter sind beispielsweise zyklische Acetale und Ketale gemeint, die u. a. als Schutzgruppe für die beiden endständigen Hydroxylfunktionen (in der 5- und 6-Position) der Ascorbinsäure eingesetzt werden. Ein bevorzugtes heterozyklisches System ist u.a. der 5,6-Isopropylidenrest, der durch Umsetzung der beiden freien Hydroxylgruppen in der 5- und 6-Position mit Aceton gebildet wird.

Als Kationen für R³ kommen NH₄⁺ sowie Vertreter aus der Gruppe der Alkali- und Erdalkalimetalle in Frage, bevorzugt NH₄⁺, Na, K, Li, Ca und Mg, besonders bevorzugt Na, K und Mg.

Unter dem Begriff Ascorbylsorbate sind sowohl Sorbinsäurederivate der L- als auch der D-Ascorbinsäure (Isoascorbinsäure), bevorzugt der L-Ascorbinsäure zu verstehen.

Vorteilhaft an den Ascorbylsorbaten der Formel I ist, daß der stabilisierende Sorbinsäurerest an der Ascorbinsäure selber eine Wirkgruppe ist, die ebenfalls als Radikalquencher dient.

Ebenso wie mit Tocopherylsorbat läßt sich mit den Ascorbylsorbaten der Formel I ein guter Schutz vor freien Radikalen, wie z.B. durch UV-Strahlung hervorgerufen, erzielen. Tocopherol läßt sich jedoch nur mit einem Sorbinsäurerest verknüpfen. Aufgrund des hohen Molekulargewichtes des Tocopherols im Gegensatz zur Ascorbinsäure ist eine Verknüpfung von Sorbinsäure mit Ascorbinsäure vorteilhaft, da der Wirkstoffanteil pro Molgewicht höher ist. Desweiteren lassen sich an der Ascorbinsäure bis zu vier Sorbinsäurereste anknüpfen, was die radikalquenchende Wirkung weiter erhöht. Außer der Sorbinsäure lassen sich noch weitere Reste, wie z.B. Phosphat, Palmitat, an der Ascorbinsäure anbringen, womit sich Lipophilie und Löslichkeit der Verbindung beeinflussen lassen. Die erfindungsgemäße 2-O-Sorbinoyl-L-Ascorbinsäure ist wasserlöslich, die 6-O-Palmitoyl-2-O-sorbinoyl-L-ascorbinsäure ist öllöslich. Damit ist es möglich, die erfindungsgemäßen Ascorbylsorbate in sämtlichen gewünschten kosmetischen Formulierungen einzuarbeiten. Außerdem läßt sich durch die unterschiedliche Lipophilie der Verbindungen I die Penetration in die Haut steuern. Durch Formulieren einer Mischung von mehreren Ascorbylsorbaten unterschiedlicher Lipophilie läßt sich eine sehr breite Hautschicht vor UV-Strahlung schützen.

Die erfindungsgemäßen stabilen Ascorbylsorbate der Formel I eignen sich somit hervorragend als Wirkstoffe für kosmetische und pharmazeutische Zubereitungen.

So zeichnen sich die Verbindungen u.a. dadurch aus, daß man durch Variation der Reste R¹ und R² die Lipophilie der Ascorbinsäurederivate gezielt einstellen kann. Je nach Anforderung bei der Formulierung kosmetischer und pharmazeutischer Zubereitungen stehen dem Anwendungstechniker somit eine breite Palette stabiler Vitamin C-Derivate zur Verfügung. Besonders die Trisorbate und die Fettsäureester von Vitamin-C-2-monosorbat lassen sich aufgrund ihrer guten Öllöslichkeit sehr gut in Zubereitungen wie beispielsweise Salben, Lotionen, Gelen oder Emulsionen einarbeiten.

Demgemäß sind auch Gegenstand der vorliegenden Erfindung kosmetische und pharmazeutische Zubereitungen, enthaltend eine wirksame Menge mindestens einer der Verbindungen der Formel I sowie übliche kosmetische und pharmazeutische Hilfs- und Zusatzstoffe.

Die o.g. Zubereitungen können die Verbindungen der Formel I in Anteilen von 0,01 bis 10 Gew.-%, vorzugswiese 0,1 bis 8 Gew.-%, besonders bevorzugt 0,5 bis 5 Gew.-%, bezogen auf die Gesamtmenge der kosmetischen und pharmazetischen Zubereitung, enthalten.

Eingesetzt werden können die Ascorbinsäurederivate der Formel I u.a. in allen kosmetischen und pharmazeutischen Zubereitungen, die neben Wasser auch noch Emulgatoren, Stabilisatoren, natürliche Öle, kosmetische Öle, Fette , Wachse, Siliconöle, Siliconölderivate, Solubilisatoren, Lichtschutzmittel, Wirkstoffe, Feuchthaltemittel, Konsitenzgeber, Gelbildner, Antioxidantien, Konservierungsmittel enthalten.

Als Emulgatoren können z.B. folgende Substanzen verwendet werden:

Polyglycerinfettsäureester, Ethoxylate von Fettsäuren, Sorbitanfettsäureester, Phosphorsäureester von Fettsäuren, Phospholipide, Glycerinmonostearat und Glycerinmonostearat selbstemulgierend.

Unter Stabilisatoren versteht man:

Magnesium und Aluminiumsalze von Fettsäuren, Komlexbildner wie EDTA, NTA, MGDA, Antioxidantien wie BHT, BHA, alpha Tocopherol, Gallsäure und ihre Salze und Ester.

Natürliche Öle sind z.B. Jojobaöl, Sonnenblumenöl, Erdnußöl, Mandelöl, Avocadoöl, Macadamianußöl, Rizinußöl, Maiskeimöl, Traubenkernöl.

Kosmetische Öle sind z.B.Isopropylester von Fettsäuren ganz besonders Isopropylstearat, Isopropylpalmitat, Isopropylisostearat, Isopropylmyristat, Isopropyllaurat, Paraffinöl, Neutralöl.

Kosmetische Wirkstoffe sind z.B. Panthenol, Bisabolol, a-Tocopherol, a-Tocopherolacetat, Aloe Vera, Algenextrakt, Hyaluronsäure, Retinol und Retinylester, Phytantriol, Panthenylethylether, Ferulasäure.

Lichtschutzmittel, die alleine oder als Gemisch zusammen mit den Verbindungen der Formel I verwendet werden können sind z.B.

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4 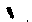 -methoxy-dibenzoylmethan | 70356-09-1 |
| 29 | 2,2 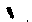 ,4,4 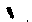 -Tetrahydroxybenzophenon | 131-55-5 |

Die Ascorbylsorbate der Formel I sind auch für tensidischen Formulierungen geeignet.

So können problemlos Haarspülungen, Shampoos, Schäume mit stabilem Vitamin C der Formel I hergestellt werden.

Die Kombination von anionischen mit kationischen Tensiden schränkt den Einsatz in Kosmetikprodukten nicht ein.

Die Synthese der Ascorbylsorbate der Formel I erfolgt in an sich bekannter Weise durch basenkatalysierte Umsetzung der Ascorbinsäurederivate der Formel II mit Sorbinsäure oder Sorbinsäurederivaten, insbesondere mit Sorbinsäurechlorid. Die Herstellung erfolgt analog den bekannten Verfahren zur Acylierung von Ascorbinsäure gemäß B. M. Tolberg et al. Ann., N. Y. Acad. Sci., 258 (1975) 48-69.

In den nachfolgenden Beispielen wird die Herstellung der erfindungsgemäßen Ascorbinsäurederivate der Formel I sowie die diese Verbindungen enthaltenden kosmetischen Formulierungen näher erläutert.

### Beispiel 1

### 2,5,6-O-Trisorbinoyl-L-ascorbinsäure

In einem 100 ml 3-Halskolben wurden 2 g (11,36 mmol; 1 eq.) L-Ascorbinsäure in 50 ml Dichlormethan suspendiert. Nacheinander wurden bei - 15 °C 0,28 g (2,27 mmol; 0,2 eq.) N,N'-Dimethylaminopyridin, 5,2 ml (37,49 mmol; 3,3 eq.) Triethylamin und zuletzt langsam 4,89 g (37,49 mmol; 3,3 eq.) Sorbinsäurechlorid hinzugetropft. Anschließend wurde bei 0 °C über Nacht gerührt. Das ausgefallene Salz wurde abfiltriert und das Filtrat mit 1 M HCl extrahiert. Die organische Phase wurde mit gesättigter NaCl-Lösung extrahiert, über MgSO₄ getrocknet und anschließend eingeengt.
Ausbeute: 4,4 g (85 % Ausbeute)

### Beispiel 2

### 5,6-0-Isopropyliden-2-0-sorbinoyl-L-ascorbinsäure

In einem 2l 4-Halskolben wurden 100,0 g (462,5 mmol; 1 eq.) 5,6-0-Isopropyliden-L-ascorbinsäure in einer N₂-Atmosphäre in 900 ml Dichlormethan gelöst. Nacheinander wurden 11,30 g (92,51 mmol; 0,2 eq.) Dimethylaminopyridin und 64.0 ml (462,5 mmol; 1 eq.) Triethylamin hinzugegeben. Anschließend wurden bei - 15 °C 60,37 g (462,5 mmol; 1 eq.) Sorbinsäurechlorid, gelöst in 100 ml Dichlormethan, langsam hinzugetropft, wobei die Innentemperatur - 10 °C nicht überschritt. Anschließend wurde 10 Stunden bei Raumtemperatur gerührt. Der Ansatz wurde mit 0.1 M HCl und anschließend H₂O gewaschen. Danach erfolgte eine Extraktion mit gesättigter wäßriger Natriumhydrogencarbonat-Lösung. Die organische Phase wurde abgetrennt und die wäßrige Phase mit konzentrierter Salzsäure bis pH 1 versetzt. Dabei fiel ein weißer Niederschlag aus, der im Ölpumpenvakuum getrocknet wurde.
Ausbeute: 83 g (58 % Ausbeute)

### Beispiel 3

### 2-0-Sorbinoyl-L-ascorbinsäure

In einem 250 ml 2-Halskolben wurden 5 g (0,012 5,6-O-Isopropyliden-2-O-sorbinoyl-L-ascorbinsäure in 160 ml Essigsäure (60 %) gelöst und 4 Stunden bei 50 °C gerührt. Anschließend wird die Lösung am Rotationsverdampfer eingeengt und mit Toluol codestilliert. Das Rohprodukt wurde an Kieselgel gereinigt (Laufmittel Dichlormethan/Methanol 5:1).
Ausbeute: 3,1 g (96 % Ausbeute)

### Beispiel 4

### 6-0-Palmitoyl-2-0-sorbinoyl-L-ascorbinsäure

In einem 11 4-Halskolben wurden 50 g (120 mmol; 1 eq.) 6-0-Palmitoyl-L-ascorbinsäure in 400 ml Pyridin gelöst. 23,40 g (130 mmol; 1,1 eq.) Sorbinsäurechlorid, gelöst in 30 ml Dichlormethan, wurden bei einer Innentemperatur von - 15 °C langsam hinzugetropft. Dabei wurde darauf geachtet, daß die Innentemperatur - 10 °C nicht überstieg. Der Ansatz wurde auf Raumtemperatur gebracht und noch 2 Stunden gerührt. Am Rotationsverdampfer wurde die Reaktionslösung eingeengt und anschließend mit Toluol codestilliert. Der Rückstand wurde in wenig Dichlormethan aufgenommen und mit 1 M HCl extrahiert. Die organische Phase wurde etwas eingeengt und mit einem Überschuß n-Hexan versetzt. Es fiel ein weißer Niederschlag aus, der im Ölpumpenvakuum getrocknet wurde.
Ausbeute: 28 g (46 % Ausbeute)

### Kosmetische Zubereitungen

### Beispiel 5

| Zusammensetzung für fettfreies Sonnenschutz-Gel | |
|---|---|
| Massengehalt (Gew.-%) | |
| 0,40 | Acrylate/C₁₀-C₃₀ Alkylacrylate crosspolymer |
| 0,25 | Hydroxyethyl cellulose |
| 8,00 | Methoxycinnamic-octylester |
| 1,00 | 4-Methylbenzylidene camphor |
| 0,50 | 6-O-Palmitoyl-2-O-sorbinoyl-L-ascorbinsäure |
| 0,20 | Disodium EDTA |
| 5,00 | Glycerin |
| 0,15 | Fragrance |
| 0,30 | Imidazolydinyl urea |
| 0,25 | Sodium methylparaben |
| 0,15 | Sodium propylparaben |
| 5,00 | PEG-25 PABA |
| 0,10 | Sodiumhydroxide |
| ad 100 | Water |

### Beispiel 6

| Zusammensetzung für Feuchtigkeitscreme | |
|---|---|
| Massengehalt (Gew.-%) | |
| 6,00 | PEG-7-Hydrogenated castor oil |
| 5,00 | Isopropylpalmitate |
| 6,00 | Mineral oil |
| 5,00 | Jojoba oil |
| 5,00 | Almond oil |
| 0,50 | Tocopherylacetate |
| 2,00 | 2,5,6-O-Trisorbinoyl-L-ascorbinsäure |
| 0,60 | Magnesiumstearate |
| 2,00 | PEG-45 / Dodecyl glycol copolymer |
| 5,00 | Glycerin |
| 0,25 | Methylparaben |
| 0,15 | Propylparaben |
| 5,00 | Imidazolydinyl urea |
| 0,15 | Fragrance |
| 0,20 | Disodium EDTA |
| ad 100 | Water |

### Beispiel 7

| Zusammensetzung für Feuchtigkeitscreme | |
|---|---|
| Massengehalt (Gew.-%) | |
| 6,00 | PEG-7-Hydrogenated castor oil |
| 5,00 | Isopropylpalmitate |
| 6,00 | Mineral oil |
| 5,00 | Jojoba oil |
| 5,00 | Almond oil |
| 0,50 | Tocopherylacetate |
| 2,00 | 6-O-Palmitoyl-2-O-sorbinoyl-L-ascorbinsäure |
| 0,60 | Magnesiumstearate |
| 2,00 | PEG-45/Dodecyl glycol copolymer |
| 5,00 | Glycerin |
| 0,25 | Methylparaben |
| 0,15 | Propylparaben |
| 5,00 | Imidazolydinyl urea |
| 0,15 | Fragrance |
| 0,20 | Disodium EDTA |
| ad 100 | Water |

### Beispiel 8

| Zusammensetzung für Nachtcreme ohne Konservierungsmittel | |
|---|---|
| Massengehalt (Gew.-%) | |
| 5,00 | PEG-7-Hydrogenated castor oil |
| 4,00 | Isopropylpalmitate |
| 4,00 | Caprylic acid/Caprinate triglyceride |
| 3,00 | 6-O-Palmitoyl-2-O-sorbinoyl-L-ascorbinsäure |
| 1,50 | PEG-45/Dodecyl glycol copolymer |
| 0,50 | Magnesiumstearate |
| 1,50 | Dimethicon |
| 4,00 | 1,2 Propyleneglycol |
| 4,00 | Glycerin |
| 8,00 | 611 Alcohol |
| 2,00 | Kollagen |
| 0,15 | Fragrance |
| ad 100 | Water |

### Beispiel 9

| Zusammensetzung für Antifaltencreme | |
|---|---|
| Massengehalt (Gew.-%) | |
| 6,00 | PEG-7-Hydrogenated castor oil |
| 5,00 | Isopropylpalmitate |
| 10,00 | Mineral oil |
| 3,00 | Caprylic acid/Caprate triglyceride |
| 0,60 | Magnesiumstearate |
| 1,00 | 2-O-Sorbinoyl-L-ascorbinsäure |
| 1,50 | Tocopherylacetate |
| 2,00 | PEG-45/Dodecyl Glycol Copolymer |
| 0,05 | Tocopherol |
| 0,20 | Retinol |
| 0,30 | Glycerin |
| 0,70 | Magnesiumsulfate |
| 0,25 | Methylparaben |
| 0,15 | Propylparaben |
| 0,20 | Sodiumascorbylmonophosphat |
| 0,10 | α-Tocopherol |
| 0,10 | Ascorbylpalmitate |
| 0,15 | Fragrance |
| ad 100 | Water |

### Beispiel 10

| Zusammensetzung für Antifaltencreme | |
|---|---|
| Massengehalt (Gew.-%) | |
| 6,00 | PEG-7-Hydrogenated castor oil |
| 5,00 | Isopropylpalmitate |
| 10,00 | Mineral oil |
| 3,00 | Caprylic acid/Caprate triglyceride |
| 0,60 | Magnesiumstearate |
| 1,00 | 5,6-O-Isopropyliden-2-O-sorbinoyl-L-ascorbinsäure |
| 1,50 | Tocopherylacetate |
| 2,00 | PEG-45/Dodecyl Glycol Copolymer |
| 0,05 | Tocopherol |
| 0,20 | Retinol |
| 0,30 | Glycerin |
| 0,70 | Magnesiumsulfate |
| 0,25 | Methylparaben |
| 0,15 | Propylparaben |
| 0,20 | Sodiumascorbylmonophosphat |
| 0,10 | α-Tocopherol |
| 0,10 | Ascorbylpalmitate |
| 0,15 | Fragrance |
| ad 100 | Water |

### Beispiel 11

| Zusammensetzung für Feuchtigkeits Tagescreme | |
|---|---|
| Massengehalt (Gew.-%) | |
| 2,00 | Ceteareth/6 |
| 2,00 | Ceteareth/25 |
| 10,00 | Mineral oil |
| 3,00 | Caprylic acid/Caprate triglyceride |
| 3,00 | Isostearic acid |
| 3,00 | 6-O-Palmitoyl-2-O-sorbinoyl-L-ascorbinsäure |
| 1,50 | Tocopherylacetate |
| 2,00 | D-Panthenol USP |
| 0,05 | Tocopherol |
| 0,20 | Retinol |
| 0,30 | Glycerin |
| 0,15 | Dibromocyanobutane |
| 0,20 | Sodiumascorbylmonophosphate |
| 0,10 | α-Tocopherol |
| 0,10 | Ascorbylpalmitate |
| 0,15 | Fragrance |
| ad 100 | Water |

### Beispiel 12

| Zusammensetzung für Feuchtigkeits Tagescreme | |
|---|---|
| Massengehalt (Gew.-%) | |
| 2,00 | Ceteareth/6 |
| 2,00 | Ceteareth/25 |
| 10,00 | Mineral oil |
| 3,00 | Caprylic acid/Caprate triglyceride |
| 3,00 | Isostearic acid |
| 3,00 | 2-O-Sorbinoyl-L-ascorbinsäure |
| 1,50 | Tocopherylacetate |
| 2,00 | D-Panthenol USP |
| 0,05 | Tocopherol |
| 0,20 | Retinol |
| 0,30 | Glycerin |
| 0,15 | Dibromocyanobutane |
| 0,20 | Sodiumascorbylmonophosphate |
| 0,10 | α-Tocopherol |
| 0,10 | Ascorbylpalmitate |
| 0,15 | Fragrance |
| ad 100 | Water |

### Beispiel 13

| Zusammensetzung für fettfreies Gel | |
|---|---|
| Massengehalt (Gew.-%) | |
| 8,00 | Octyl Methoxycinnamate |
| 7,00 | Titanium Dioxide |
| 2,00 | 2-O-Sorbinoyl-L-ascorbinsäure |
| 5,00 | Glycerin |
| 5,00 | PEG-25 PABA |
| 1,00 | 4-Methylbenzylidene Camphor |
| 0,40 | Acrylates/C10-C30 Alkyl Acrylate Crosspolymer |
| 0,30 | Imidazolidinyl urea |
| 0,25 | Hydroxyethylcellulose |
| 0,25 | Sodium methylparaben |
| 0,20 | Disodium EDTA |
| 0,15 | Fragrance |
| 0,15 | Sodium propylparaben |
| 0,10 | Sodiumhydroxide |
| ad 100 | Water |

### Beispiel 14

| Zusammensetzung für Sonnencreme (LSF 20) | |
|---|---|
| Massengehalt (Gew.-%) | |
| 8,00 | Octyl methoxycinnamate |
| 8,00 | Titaniumdioxide |
| 6,00 | PEG-7-Hydrogenated castor oil |
| 2,00 | 2-O-Sorbinoyl-L-ascorbinsäure |
| 6,00 | Mineral oil |
| 5,00 | Zinc oxide |
| 5,00 | Isopropylpalmitate |
| 5,00 | Imidazolidinyl urea |
| 3,00 | Jojoba oil |
| 2,00 | PEG-45/Dodecyl glycol copolymer |
| 1,00 | 4-Methylbenzylidene camphor |
| 0,60 | Magnesiumstearate |
| 0,50 | Tocopherylacetate |
| 0,25 | Methylparaben |
| 0,20 | Disodium EDTA |
| 0,15 | Propylparaben |
| ad 100 | Water |

### Beispiel 15

| Zusammensetzung für Sonnencreme (wasserfest) | |
|---|---|
| Massengehalt (Gew.-%) | |
| 8,00 | Octyl methoxycinnamate |
| 5,00 | PEG-7-Hydrogenated castor oil |
| 5,00 | Propylene glycol |
| 4,00 | Isopropyl palmitate |
| 4,00 | Caprylic/capric triglyceride |
| 3,00 | 6-O-Palmitoyl-2-O-sorbinoyl-L-ascorbinsäure |
| 4,00 | Glycerin |
| 3,00 | Jojoba oil |
| 2,00 | 4-Methylbenzylidene camphor |
| 2,00 | Titanium dioxide |
| 1,50 | PEG-45/Dodecyl glycol copolymer |
| 1,50 | Dimethicone |
| 0,70 | Maghesiumsulfate |
| 0,50 | Magnesiumstearate |
| 0,15 | Fragrance |
| ad 100 | Water |

### Beispiel 16

| Zusammensetzung für Sonnenmilch (LSF 6) | |
|---|---|
| Massengehalt (Gew.-%) | |
| 10,00 | Mineral oil |
| 6,00 | PEG-7-Hydrogenated castor oil |
| 5,00 | Isopropylpalmitate |
| 3,50 | Octyl methoxycinnamate |
| 2,00 | 2,5,6-Trisorbinoyl-L-ascorbinsäure |
| 3,00 | Caprylic/capric triglyceride |
| 3,00 | Jojoba oil |
| 2,00 | PEG-45/Dodecyl glycol copolymer |
| 0,70 | Magnesiumsulfate |
| 0,60 | Magnesiumstearate |
| 0,50 | Tocopherylacetate |
| 0,30 | Glycerin |
| 0,25 | Methylparaben |
| 0,15 | Propylparaben |
| 0,05 | Tocopherol |
| ad 100 | Water |

### Beispiel 17

| Zusammensetzung für Lippenstift | |
|---|---|
| Massengehalt (Gew.-%) | |
| 10,00 | Glycerin |
| 10,00 | Titanium dioxide |
| 2,00 | 2,5,6-Trisorbinoyl-L-ascorbinsäure |
| 8,00 | Octyl methoxycinnamate |
| 5,00 | Zinc oxide |
| 4,00 | Castor oil |
| 4,00 | Pentaerythrithyl Stearate/caprate/caprylate adipate |
| 3,00 | Glycerylstearate SE |
| 2,00 | Bees ax |
| 2,00 | Microcrystalline Wax |
| 2,00 | Quaternium-18 Bentonite |
| 1,50 | PEG-45/Dodecyl glycol copolymer |
| ad 100 | Eucerinum anhydricum |

### Beispiel 18

| Zusammensetzung für Sunblock mit Mikropigmenten | |
|---|---|
| Massengehalt (Gew.-%) | |
| 10,00 | Parsol MCX octyl methoxycinnamate |
| 6,00 | PEG-7-Hydrogenated castor oil |
| 6,00 | Titanium dioxide |
| 2,00 | 5,6-O-Isopropyliden-2-O-sorbinoyl-L-ascorbinsäure |
| 5,00 | Mineral oil |
| 5,00 | Isoamyl p-Methoxycinnamate |
| 5,00 | Propylene glycol |
| 3,00 | Jojoba oil |
| 3,00 | 4-Methylbenzylidene camphor |
| 2,00 | PEG-45/Dodecyl glycol copolymer |
| 1,00 | Butyl methoxydibenzoylmethane |
| 1,00 | Dimethicone |
| 0,50 | PEG-40-Hydrogenated castor oil |
| 0,50 | Tocopherylacetate |
| 0,50 | Phenoxyethanol |
| 0,20 | EDTA |
| ad 100 | Water |

## Patentansprüche

1. Ascorbylsorbate der allgemeinen Formel I, 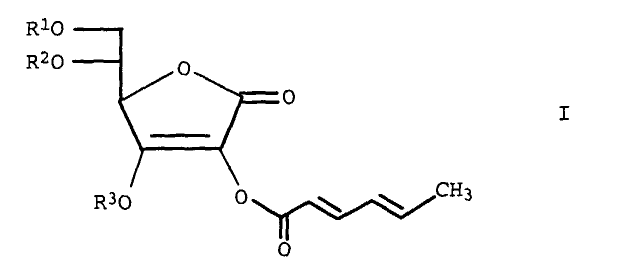 in der die Variablen unabhängig voneinander folgende Bedeutung haben:
R¹ und R²
Wasserstoff, C₁-C₁₂-Alkyl, C₁-C₂₀-Acyl;
wobei R¹ und R² gemeinsam mit den Sauerstoffatomen an die sie gebunden sind und den an den Sauerstoffatomen gebundenen Kohlenstoffatomen einen gegebenenfalls substituierten Heterozyklus bilden können;
R³ Wasserstoff oder ein Kation, ausgewählt aus der Gruppe, bestehend aus NH₄⁺, Alkali- und Erdalkalimetallkationen.

2. Ascorbylsorbate der Formel I gemäß Anspruch 1, in der die Variablen unabhängig voneinander folgende Bedeutung haben:
R¹ und R²
Wasserstoff, C₆-C₂₀-Acyl;
R³ Wasserstoff oder ein Kation, ausgewählt aus der Gruppe, bestehend aus NH₄⁺, Alkali- und Erdalkalimetallkationen.

3. Ascorbylsorbate der Formel I gemäß den Ansprüchen 1 und 2, in der die Variablen unabhängig voneinander folgende Bedeutung haben:
R¹ und R²
Wasserstoff, 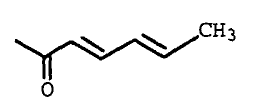
R³ Wasserstoff oder ein Kation, ausgewählt aus der Gruppe, bestehend aus NH₄⁺, Alkali- und Erdalkalimetallkationen.

4. Verfahren zur Herstellung der Ascorbylsorbate der Formel I gemäß den Ansprüchen 1 bis 3, dadurch gekennzeichnet, daß man Ascorbinsäure oder Ascorbinsäurederivate der Formel II, in der die Substituenten R¹ bis R³ die in den Ansprüchen 1 bis 3 genannte Bedeutung haben mit Sorbinsäure oder Sorbinsäurederivaten umsetzt.

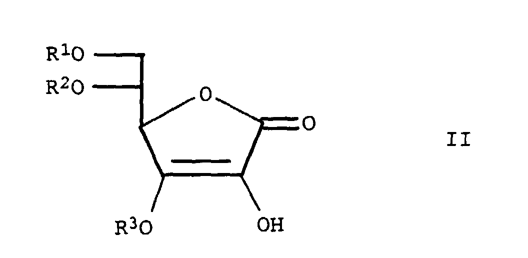

5. Verfahren nach Anspruch 4, dadurch gekennzeichnet, daß man Ascorbinsäure oder Ascorbinsäurederivate der Formel II mit Sorbinsäurechlorid umsetzt.

6. Verwendung von Ascorbylsorbaten der allgemeinen Formel I gemäß den Ansprüchen 1 bis 3 für kosmetische und pharmazeutische Zubereitungen.

7. Verwendung von Ascorbylsorbaten nach Anspruch 6 als Antioxidans in kosmetischen und pharmazeutischen Formulierungen.

8. Kosmetische und pharmazeutische Zubereitungen, enthaltend eine wirksame Menge mindestens einer der Verbindungen der Formel I gemäß den Ansprüchen 1 bis 3 sowie übliche kosmetische und pharmazeutische Hilfs- und Zusatzstoffe.
